# EUROPEAN PATENT APPLICATION

(11) **EP 3 299 017 A1**
(43) Date of publication of application: **28.03.2018**
(21) Application number: 16020346.9
(22) Date of filing: 23.09.2016
(51) Int. Cl.: A61K 31/4439, A61K 9/10, A61K 47/14, A61K 47/44

(54) **FORMULATION**

(71) Applicant: The Boots Company PLC, Nottingham NG2 3AA (GB)
(72) Inventor: Sherry, Robert Arthur, Toton, NOTTINGHAM NG9 6LF (GB); Barfield, John Gerard, Thorneywood, NOTTINGHAM NG3 3AW (GB); Tang, Weng Sam, Cavendish Crescent South, NOTTINGHAM NG7 1ED (GB)
(74) Representative: Goodier, Claire-Louise

(57) **Abstract**

According to the present invention, there is provided a liquid formulation for oral administration comprising an active pharmaceutical ingredient dispersed in a pharmaceutically acceptable oily carrier, the oily carrier comprising a major proportion of a triglyceride-based oil and a minor proportion of a waxy compound.

## Description

The present invention relates to a pharmaceutical formulation, in particular to an orally administered pharmaceutical formulation suitable for stabilising acid-sensitive active ingredients.

Pharmaceutical active ingredients are frequently administered orally. Such active ingredients are commonly absorbed into the bloodstream from the small intestine, and in order to reach the small intestine they must pass through the stomach without degrading. However, certain active ingredients are particularly sensitive to the stomach's highly acidic environment.

In particular, benzimidazole compounds, such as omeprazole, lansoprazole, rabeprazole and pantoprazole, which have gastric acid secretion inhibitory activity, gastric mucosa protecting activity, and are widely used as peptic ulcer treating agents,are known to be highly unstable in acidic conditions, with the result that they may degrade in the acidic environment of the stomach before they can be absorbed systemically.

To address this problem, drugs of this kind are conventionally formulated as solid dosage forms, such as tablets or capsules, comprising a pH-sensitive protective polymer that is intended to prevent the tablet or capsule from dissolving in the acidic environment found in the stomach, and hence to convey the active ingredient to the small intestine without degradation.

However, for various reasons, some individuals find it difficult or impossible to swallow tablets or capsules. This may be the case, for instance, for young children or the elderly, or for certain patients having other medical conditions. For such patients, there may be no alternative to liquid formulations that are more palatable and easier to swallow. However, formulation of an active ingredient into a liquid for oral administration increases the potential for the active ingredient to be adversely affected by the acidity of the stomach, or to interact with other components of the formulation in an adverse way.

Oral liquids containing omeprazole are known. However, these are produced as short shelf-life formulations and contain significant amounts of a buffering component such as sodium bicarbonate to provide some limited protection during gastric transit. These formulations must be stored in a refrigerator and have a shelf-life typically of only one to three months. Other formulations have been proposed that contain pH-sensitive polymers such as those used in capsules, but those ingredients may react with omeprazole and the like and are therefore unsuitable for use with drugs of that class.

Powder formulations are also known, but are inconvenient due to the need to dissolve the powder in water immediately prior to ingestion. Also, these formulations contain significant quantities of pH buffering agents, typically sodium bicarbonate, which may be unsuitable for those on restricted or low sodium diets.

There is therefore a need for a palatable and stable oral liquid dosage form that is capable of inhibiting or preventing the degradation of acid-sensitive active ingredients during transit through the stomach.

There has now been devised a pharmaceutical formulation that overcomes or substantially mitigates the above-mentioned and/or other disadvantages of the prior art.

According to a first aspect of the present invention, there is provided a liquid formulation for oral administration comprising an active pharmaceutical ingredient dispersed in a pharmaceutically acceptable oily carrier, the oily carrier comprising a major proportion of a triglyceride-based oil and a minor proportion of a waxy compound.

It has surprisingly been found that by using such an approach, a stable gastro-protective oral liquid formulation is produced. The formulation is particularly advantageous as it prevents the active ingredient from being released in the acidic environment found in the stomach at a pH of approximately 1.5-4.5, but allows the drug to be released after passing through the stomach, ie at the approximately neutral pH found in the small intestine. The present formulation enables the release of the active ingredient to be controlled such that significant release of the active ingredient does not occur until after the formulation has passed through the stomach. Thus, the active ingredient is not significantly degraded before it can be absorbed in the patient's small intestine.

The oily carrier comprises a major proportion of triglyceride-based oil. By "triglyceride-based oil" is meant an oil that is liquid at ambient temperatures and which is made up entirely or largely of triglyceride molecules. Ambient temperatures in this context mean temperatures of the surroundings in which the formulation of the invention is likely to be dispensed in normal use; such temperatures will typically be between 5°C and 40°C, or between 10°C and 30°C. Examples of oils made up entirely or largely of triglyceride molecules include vegetable oils, as well as analogous synthetic or semi-synthetic materials. The triglyceride-based oil may be a mixture of such materials.

By a "major proportion" is meant that the triglyceride-based oil forms the majority of the carrier, ie at least 50% of the carrier by weight. More preferably the triglyceride-based oil constitutes at least 60%, at least 70%, at least 80%, at least 90%, or at least 95% of the carrier by weight. Most preferably the triglyceride-based oil constitutes at least 97% of the carrier. The triglyceride-based oil will normally account for less than 99% by weight of the carrier. Thus, the triglyceride-based oil may account for about 98% by weight of the carrier.

Vegetable oils that may be used as, or as part of, the triglyceride-based oil include, without limitation, castor oil, coconut oil, corn oil, ground nut oil, olive oil, palm oil, rapeseed oil, soybean oil, arachis oil, and sunflower oil.

Other materials that may be used as, or as part of, the triglyceride-based oil are purified or fractionated triglycerides that may be obtained from vegetable oils or other sources. Such triglycerides may be those referred to as medium chain triglycerides or those referred to as long chain triglycerides. By medium-chain triglycerides (MCTs) is meant triglycerides containing fatty acid residues of 6-12 carbon atoms in length. By long-chain triglycerides (LCTs) is meant triglycerides containing acid residues of more than 12 carbon atoms in length, or more than 16 carbon atoms in length.

The triglyceride-based oil may be a vegetable oil that contains predominantly long chain triglycerides, such as sunflower oil (which comprises high proportions of oleic and linoleic triglycerides) or corn oil (which comprises high proportions of linoleic triglycerides). More preferably, however, the triglyceride-based oil comprises predominantly medium-chain triglycerides, for example caprylic/capric triglycerides, available for example under the trade names Miglyol 812, Crodamol GTCC, or Kollisolv MCT60/MCT70. Medium-chain triglycerides are advantageous because they are bland in flavour compared to other fats and are also more polar than long-chain triglycerides and thus certain active ingredients may be more soluble in the carrier if a medium-chain triglyceride is chosen. Medium-chain triglycerides are also easily metabolised by the human body and are therefore generally suitable for oral ingestion.

The aliphatic chains of the triglycerides may be saturated or unsaturated. Preferably the triglycerides contain mostly aliphatic chains that are saturated. In general, the triglyceride-based oil will contain mixtures of triglycerides with fatty acid residues of differing chain lengths and/or levels of unsaturation. The triglyceride-based oil may also contain minor proportions of mono- and/or di-glycerides, as well as minor amounts of other components such as free fatty acids and other impurities. Oils of natural origin may be particularly heterogeneous; synthetic or semi-synthetic materials may be more uniform in their composition.

By "waxy compound" is meant an organic compound that is a hydrophobic, malleable solid at and near ambient temperatures. Examples include higher alkanes (ie hydrocarbon compounds of the formula CₙH₂ₙ₊₂, where n is at least 18, more commonly at least 20 or at least 24, and n is typically up to 40, or up to 60) and lipids, including mono-, di- and tri-glycerides and phospholipids. Waxes typically have melting points above about 40°C. Waxy compounds are insoluble in water (by which is meant having a solubility in distilled water of less than about 1 gram per 100 mL, and typically less than 0.5 gram or less than 0.1 gram per 100 mL), but are generally soluble in organic, nonpolar solvents.

Preferably the waxy compound is a long-chain mono- or di-glyceride or a mixture of such compounds.

By long-chain mono- or di-glycerides is meant glycerides with one or two fatty acid residues, those fatty acid residues being are greater than 12 carbon atoms in length, and preferably greater than 16 carbon atoms.

Most preferably the long-chain mono- or diglyceride contains fatty acid residues of length greater than 20 carbon atoms. Most preferably the mono- or diglyceride is glyceryl behenate. In particularly preferred embodiments, the behenate is a combination of mono- and dibehenate as found in glyceryl behenate EP/NF supplied under the tradename Compitrol 888 ATO.

Other suitable waxy compounds may include plant and animal waxes such as carnauba wax and beeswax, petrolatum waxes such as microcrystalline wax, and long chain aliphatic esters such as cetyl palmitate. Further examples include long-chain (typically C₁₂ and above) fatty acids that are solid at ambient temperature, such as palmitic acid and stearic acid, as well as esters of dicarboxylic acids such as fumaric, succinic and sebacic acid (eg dibutyl sebacate, diethyl sebacate and alkyl fumarates and alkyl succinates). Certain polyethylene glycols (PEGs) that are solid at ambient temperature may also be suitable, eg PEG6000 and analogues thereof).

Waxy compounds such as glyceryl behenate have previously been used to delay the release of drugs by forming part of a solid coating for a tablet or similar solid dosage form. However, the applicant has now surprisingly found that they can modulate the pH-sensitive release of drugs from a liquid formulation.

The waxy compound makes up a minor proportion of the oily carrier, ie it accounts for less than 50% of the carrier by weight. More preferably the waxy compound comprises less than 40%, less than 30%, less than 20%, less than 10%, or less than 5% by weight of the carrier. Most preferably the waxy compound comprises less than 3% of the carrier by weight. In particularly preferred embodiments, the waxy compound comprises approximately 2% w/w of the carrier.

The active ingredient is dispersed in the oily carrier. The active ingredient may be dissolved in the oily carrier, either wholly or partially. More commonly, however, the active ingredient will be suspended in the oily carrier. In such cases, the active ingredient is preferably present in finely divided form, eg in the form of particles having an average particle size in the range 1-100µm.

The invention is of greatest utility in relation to active pharmaceutical ingredients that are acid-sensitive, ie active pharmaceutical ingredients that are altered on contact with an acidic environment.

The invention is suitable for stabilising acid-unstable compounds generally but is particularly suitable for stabilising benzimidazole compounds (proton pump inhibitors) that are known to be unstable in the acidic conditions found in the stomach. Examples of such active ingredients are omeprazole, lansoprazole, dexlansoprazole, esomeprazole, pantoprazole, rabeprazole and ilaprazole. The active ingredient will most usually be a single drug compound, but may be a mixture of two or more drug compounds.

The concentration of the active pharmaceutical ingredient will depend on the required dose and the amount of the substance that can be put into solution or suspension in the formulation. Where the active pharmaceutical ingredient is a proton pump inhibitor, the proton pump inhibitor may be present at a concentration in the range from 1mg/ml to 10 mg/ml, for instance 2 mg/ml, 4mg/ml or 8mg/ml. For a dose of 5 ml, those concentrations correspond to a unit dose of 10mg, 20mg or 40mg.

The formulation according to the invention is also beneficial in that it has an acceptable shelf life. By that is meant that, when kept under normal storage conditions, a packaged formulation according to the invention is stable for at least three months, and more preferably for at least six months or for at least twelve months. In this context, "stable" means that at least 90%, and more preferably at least 95% or at least 98% by weight of the active ingredient remains in an active form in the formulation over the stated time period.

The formulation may be put up in unit dose form. For instance, the formulation may be packaged in a sachet containing, for example, a unit dose of from 1ml to 20ml of the formulation. In currently preferred embodiments of the invention, the dose is 5ml. Alternatively, the formulation of the invention may be put up in a bulk form from which individual doses may be dispensed as required. The formulation may, for instance, be packaged in a bottle or the like, from which individual doses may be dispensed by pouring into a spoon or other measuring vessel, of from which doses may be dispensed by a metering mechanism, such as a metering pump, or a dosing device, such as a syringe.

The formulation is intended to protect an acid-sensitive drug from the harsh acidity of the stomach but conversely it is also contemplated that the technology could be suitable to protect the stomach from adverse effects caused by a drug, for example in the case of a drug that causes irritation to the stomach lining. Examples of such drugs include ibuprofen and other 2-arylpropionic acids or profens, diclofenac, cyclooxygenase-2 (COX-2) inhibitors (eg celecoxib, etoricoxib, firocoxib, lumiracoxib, parecoxib, rofecoxib and valdecoxib), nitrofurantoin, alendronate, corticosteroids and sulpasalazine.

Thus, the invention further provides a method of treating a patient having or susceptible to a condition that may be ameliorated or prevented by an active pharmaceutical ingredient that is sensitive to the acidity of the patient's stomach and/or that may induce an adverse effect upon the patient's stomach, which method involves oral administration to the patient of a formulation according to the first aspect of the invention.

The formulation may comprise further additional pharmaceutically acceptable components. The additional components may be any appropriate pharmaceutical excipients that are conventionally included in oral liquids, for example flavourants to mask or improve the flavour of the active ingredient and/or the oily carrier, dispersants to keep the active ingredient in suspension, stabilisers and buffers. In particular, the formulation may comprise a pH modifier or stabiliser, for example, meglumine, calcium carbonate, sodium carbonate or magnesium carbonate.

Where the formulation comprises a pH modifier, the pH modifier may be present in the range 0.001% to 1% w/v, more preferably in the range 0.005% to 0.5% w/v.

Optionally, at least one anti-oxidant may be included. Examples of anti-oxidants include, without limitation, butylhydroxytoluene (BHT), butylhydroxyanisole (BHA), tert-butylhydroquinone (TBHQ), gallic acid esters such as propyl gallate, tocopherols such as vitamin E acetate, ascorbic acid esters such as ascorbyl palmitate and ascorbyl acetate, carnitine, and/or mixtures thereof.

The formulation may be prepared by admixing the various ingredients, with heating and/or agitation as necessary in order to achieve homogeneity. In a presently preferred process, the waxy compound is introduced to a suitable vessel and the triglyceride-based oil then added. The mixture may be heated and agitated (eg by stirring) to homogenise it. The temperature to which the mixture is heated may be below the melting point of the waxy compound. The mixture may then be cooled (for instance to around ambient temperature, eg 20-30°C) prior to addition of the active ingredient, with further agitation as necessary to achieve uniform dispersal of the active ingredient in the oily carrier. Further ingredients such as pH modifiers, anti-oxidants etc may be added at any suitable stage of the process.

Thus, according to a further aspect of the invention, there is provided a process for the preparation of a formulation as described above, which process comprises the steps of
a) dispersing the waxy compound in the triglyceride-based oil, optionally with heating and agitation, in order to form the oily carrier; and
b) dispersing the active pharmaceutical ingredient in the oily carrier.

The invention will now be described in greater detail, by way of illustration only, with reference to the following Examples and the accompanying figures, in which
Figure 1 illustrates the dissolution profile as a function of time of a formulation according to the invention (solid line) in comparison with a control formulation (broken line), where the pH is varied from 1.2 to 6.8; and
Figure 2 illustrates the dissolution profile as a function of time of a formulation according to the invention (solid line) in comparison with a control formulation (broken line), where the pH is varied from 4.5 to 6.8.

### Example 1

### Omeprazole oral liquid formulation

The composition of an omeprazole oral liquid formulation according to the invention is shown below. This is an oral liquid suspension of omeprazole.

| | |
|---|---|
| Omeprazole | 20mg/5ml |
| Glyceryl dibehenate | 2% w/v |
| Medium chain triglyceride | to 100% |

The omeprazole is present as the omeprazole sodium salt, weight corrected to 20mg/5ml omeprazole free base.

Glyceryl dibehenate is glyceryl dibehenate EP/NF also known as glyceryl behenate, trade name Compitrol 888 ATO.

The medium chain triglyceride (MCT) is Medium Chain Triglycerides Ph Eur, which is also known as caprylic/capric triglycerides, and is available under a number of trade names including Miglyol 812, Crodamol GTCC, and Kollisolv MCT60/MCT70.

The formulation was prepared as follows:
1) The glyceryl behenate was added to a suitable vessel and sufficient MCT added to form a concentrated slurry.
2) The mixture was heated to approximately 60°C, and mixed until the slurry became clear.
3) The mixture was cooled to below 25°C.
4) The omeprazole (present as sodium salt) was added and dispersed by mixing.

The formulation can be presented in a format which permits the dispensing of individual dose units of 5ml, corresponding to a dose of 20mg of the active ingredient omeprazole. In other embodiments, the concentration of omeprazole may be, for instance, any other desired figure in the range from 5 mg/5ml to 40 mg/5ml.

### Example 2

### pH-dependence of the release of omeprazole

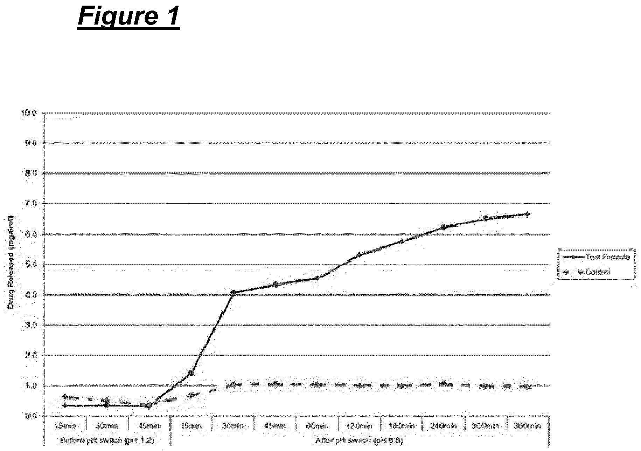

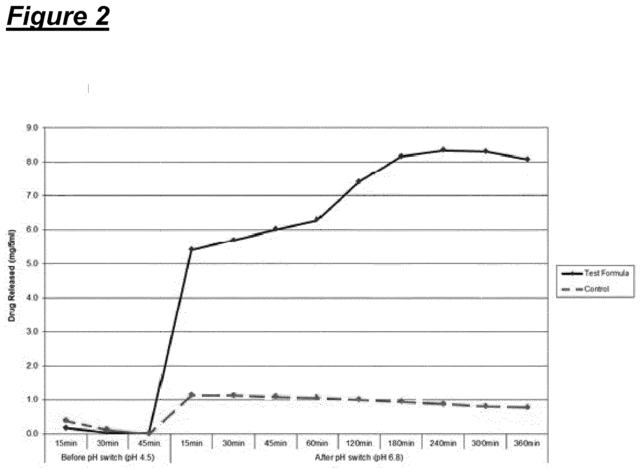

Data comparing the omeprazole oral liquid formulation as set out above with a control formulation containing omeprazole dispersed in a simple medium chain triglyceride base with no waxy compound present is illustrated in Figures 1 and 2. The data was generated using a USP dissolution apparatus (Type 2).

The materials and method used were as follows:

### Materials:

| | | |
|---|---|---|
| **Formula according to the invention (as Example 1)** | Omeprazole (as Sodium salt) | 0.4%w/v |
| | Glyceryl Dibehenate (Compritol 888 ATO) | 2.0%w/v |
| | Medium Chain Triglycerides | to 100%v/v |
| **Control** | Omeprazole (as Sodium salt) | 0.4%w/v |
| | Medium Chain Triglycerides | to 100%v/v |

### Methods:

| | |
|---|---|
| **Dissolution apparatus used** | USP Dissolution Apparatus 2 (paddle) |
| **Paddle speed** | 200rpm |
| **Volume of dissolution medium** | 700ml |
| **Volume of sample added** | 5ml |

5ml of the formulation under examination (formulation according to the invention or control) was added to 700ml of dissolution medium (simulated gastric fluid, at acidic starting pH) within the vessel of the dissolution apparatus. The formulations each contained 20mg of omeprazole, in the form of a fine suspension of solid particles. The formulation, being oily in nature, is immiscible with the aqueous dissolution medium, but the relatively high speed rotation of the paddle causes mixing of the formulation with the dissolution medium.

15, 30 and 45 minutes after addition of the formulation, aliquots of the dissolution medium were withdrawn from the vessel (using a syringe) for analysis. The pH of the dissolution medium was then adjusted and elevated to pH 6.8 (mimicing the effect of the formulation passing from the acidic environment of the stomach to the neutral environment of the small intestine). Further aliquots of dissolution medium were withdrawn at intervals, from 15 minutes to 360 minutes after the pH change.

All samples of dissolution medium were analysed by a standard HPLC method to determine the concentration of active ingredient in the sample, and hence the total amount of active ingredient released from the formulation into solution in the dissolution medium.

For the data shown in Figure 1, the starting pH of the dissolution medium was pH 1.2. The sample was exposed to acidic conditions for the first 45 minutes *(Time 0 to 45min, Before pH switch).* The pH was then adjusted and elevated to pH 6.8 and the sample then exposed to the adjusted conditions for another 360 minutes (*Time 0 to 360min, After pH switch).*

For the data shown in Figure 2, the starting pH of the dissolution medium was pH 4.5. The sample was exposed to mildly acidic condition for the first 45 minutes (*Time 0 to 45min, Before pH switch).* The pH was then adjusted and elevated to pH 6.8 and the sample was then exposed to the adjusted conditions for another 360 minutes (*Time 0 to 360min, After pH switch).*

Figures 1 and 2 illustrate the dissolution profiles for both the formulation according to the invention and the control formulation, ie the total amount of drug present in solution in the dissolution medium before and after elevation of the pH of the dissolution medium from two differing initial pH values.

From the Figures it can be seen that the release characteristics of the formulation according to the invention (Test Formula) are clearly dependent on pH. There is very limited release of the active ingredient when the pH is 1.2 or 4.5, but a rapid increase in release when the pH is adjusted to 6.8. In both cases, only a small proportion of the drug is released before the pH change, whereas after the change in pH the amount released rises rapidly to about 4-6mg and continues to rise more gradually thereafter. In comparison, the control formulation (Control), shows a steady release rate with only a small increase (approximately 0.5mg/5ml) on adjustment of the pH from 1.2 or 4.5 to 6.8.

These experiments suggest that the formulation according to the invention would show minimal drug release in the stomach environment, but would rapidly release the drug on entering the small intestine.

## Claims

1. A liquid formulation for oral administration comprising an active pharmaceutical ingredient dispersed in a pharmaceutically acceptable oily carrier, the oily carrier comprising a major proportion of a triglyceride-based oil and a minor proportion of a waxy compound.

2. A liquid formulation according to Claim 1, wherein the triglyceride-based oil comprises at least 60%, at least 70%, at least 80%, at least 90%, or at least 95% of the carrier by weight.

3. A liquid formulation according to Claim 1 or Claim 2, wherein the triglyceride-based oil comprises at least 97% of the carrier by weight.

4. A liquid formulation according to any preceding claim, wherein the triglyceride-based oil comprises predominantly medium-chain triglycerides.

5. A liquid formulation according to Claim 4, wherein the medium-chain triglycerides are capric/caprylic triglycerides.

6. A liquid formulation according to any preceding claim, wherein the waxy compound comprises less than 40%, less than 30%, less than 20%, less than 10%, less than 5%, or less than 3% by weight of the carrier.

7. A liquid formulation according to any preceding claim, wherein the waxy compound comprises approximately 2% w/w of the carrier.

8. A liquid formulation according to any preceding claim, wherein the waxy compound is a long-chain mono- or di-glyceride or a mixture of such compounds.

9. A liquid formulation according to Claim 8, wherein the long-chain mono- or di-glyceride is glyceryl behenate.

10. A liquid formulation according to Claim 9, wherein the glyceryl behenate is a mixture of glyceryl monobehenate and glyceryl dibehenate.

11. A liquid formulation according to any preceding claim, wherein the active pharmaceutical ingredient is suspended in the oily carrier.

12. A liquid formulation according to Claim 11, wherein the active pharmaceutical ingredient is present in the form of particles having an average particle size in the range 1-100µm.

13. A liquid formulation according to any preceding claim, wherein the active ingredient is an acid-sensitive active ingredient.

14. A liquid formulation according to Claim 13, wherein the active pharmaceutical ingredient is a benzimidazole compound.

15. A liquid formulation according to Claim 14, wherein the benzimidazole compound is present at a concentration in the range from 1mg/ml to 10 mg/ml.

16. A liquid formulation according to Claim 15, wherein the benzimidazole compound is present at a concentration of 2 mg/ml, 4mg/ml or 8mg/ml.

17. A liquid formulation according to any one of Claims 14 to 16, wherein the benzimidazole compound is selected from the group consisting of omeprazole, lansoprazole, dexlansoprazole, esomeprazole, pantoprazole, rabeprazole and ilaprazole

18. A liquid formulation according to any preceding claim, wherein the formulation is put up in unit dose form.

19. A liquid formulation according to Claim 18, wherein the unit dose is 5ml.

20. A liquid formulation according to any preceding claim, further comprising additional pharmaceutical excipients.

21. A liquid formulation according to Claim 20, wherein the additional pharmaceutical excipient is a pH modifier.

22. A liquid formulation according to Claim 21, wherein the pH modifier is meglumine.

23. A process for the preparation of a formulation according to any preceding claim, which process comprises the steps of
a) dispersing the waxy compound in the triglyceride-based oil, optionally with heating and agitation, in order to form the oily carrier; and
b) dispersing the active pharmaceutical ingredient in the oily carrier.

24. A process according to Claim 23, wherein the mixture formed in step a) is heated to a temperature below the melting point of the waxy compound and agitated.

25. A process according to Claim 24, wherein the oily carrier is cooled prior to addition of the active pharmaceutical ingredient.

26. A liquid formulation substantially as described herein.
